# EUROPÄISCHE PATENTSCHRIFT

(11) **EP 1 137 438 B1**
(45) Veröffentlichungstag und Bekanntmachung des Hinweises auf die Patenterteilung: **05.06.2002**
(21) Anmeldenummer: 99961041.3
(22) Anmeldetag: 01.12.1999
(51) Int. Cl.: A61K 45/06, A61P 35/00, A61P 37/00

(54) **ZUBEREITUNG MIT VERBESSERTER THERAPEUTISCHER BREITE, ENTHALTEND NUKLEOTIDSYNTHESEINHIBITOREN**
FORMULATION WITH AN IMPROVED THERAPEUTIC RANGE, CONTAINING NUCLEOTIDE SYNTHESIS INHIBITORS
FORMULATION A SPECTRE THERAPEUTIQUE ELARGI, CONTENANT DES INHIBITEURS DE LA SYNTHESE DES NUCLEOTIDES

(30) Priorität: 10.12.1998 DE 19857009
(43) Veröffentlichungstag der Anmeldung: 04.10.2001
(73) Patentinhaber: Aventis Pharma Deutschland GmbH, 65929 Frankfurt am Main (DE)
(72) Erfinder: LINDNER, Jürgen, D-35041 Marburg (DE); HAASE, Burkhard, D-65719 Hofheim (DE)
(86) Internationale Anmeldenummer: EP9909380
(87) Internationale Veröffentlichungsnummer: WO0033876

(56) Entgegenhaltungen:
- EP-A- 0 529 500
- EP-A- 0 538 783
- EP-A- 0 769 296
- WO-A-93/18776
- WO-A-98/13047
- DATABASE EMBASE [Online] ELSEVIER SCIENCE PUBLISHERS, AMSTERDAM, NL FURST D.E.: "Practical clinical pharmacology and drug interactions of low-dose methotrexate therapy in rheumatoid arthritis." retrieved from STN Database accession no. 96019438 XP002133076 & BRITISH JOURNAL OF RHEUMATOLOGY, (1995) 34/SUPPL. 2 (20-25). ,
- DATABASE CHEMABS [Online] CHEMICAL ABSTRACTS SERVICE, COLUMBUS, OHIO, US PERIS-MARTI, J. ET AL: "Inhibition of enterohepatic circulation of methotrexate by cholestiramine in rats." retrieved from STN Database accession no. 121:169641 HCA XP002133077 & EUR. J. DRUG METAB. PHARMACOKINET. (1993), (SPEC. ISSUE, PROCEEDINGS OF THE FIFTH EUROPEAN CONGRESS OF BIOPHARMACEUTICS AND PHARMACOKINETICS, 1993), 70-3 ,
- DATABASE CHEMABS [Online] CHEMICAL ABSTRACTS SERVICE, COLUMBUS, OHIO, US MCANENA, OLIVER J. ET AL: "Alteration of methotrexate metabolism in rats by administration of an elemental liquid diet. II. Reduced toxicity and improved survival using cholestyramine" retrieved from STN Database accession no. 106:207306 HCA XP002133078 & CANCER (PHILADELPHIA) (1987), 59(6), 1091-7 ,

## Beschreibung

Als Lipidsenker bei den heterozygoten familiären Hypercholesterinämie und anderen primären Hyperlipoproteinämien mit hauptsächlicher Vermehrung der LDL-Fraktion oder chologenen Diarrhöen werden stark basische Anionenaustauscher in der Therapie eingesetzt. Beispiele für geeignete Wirksubstanzen, die als Lipidsenker eingesetzt werden, sind N-(2-Aminoethyl)-N'-[2-[(2-aminoethyl)amino]ethyl]-1,2-ethandiamin Polymer mit (Chlormethyl)-oxiran, das auch als Colestipol (Colestid®) bezeichnet wird oder Colestyramin (CAS-Nr. 11 041-12-6), das ein Styroldivinylbenzyl Copolymere ist. Isoxazol- oder Crotonsäureamidderivate werden in den Patentanmeldungen EP 484 223; EP 529 500; US 4 061 767; EP 538 783 oder EP 551 230 beschrieben. Verbindungen die die Purin- oder Pyrimidinsynthese inhibieren werden als Nukleotidsyntheseinhibitoren bezeichnet (Burkhardt und Kalden; Rheumat. Int. (1997); 17: 85-90), dies sind beispielsweise Verbindungen der Formel I und/oder II, Brequinar (6-Fluor-2-(2'-fluoro[1,1'biphenyl]-4-yl)-3-methyl-4-quinolincarbonsäure), Mycophenolatmofetil (2-Morpholinoethyl-(E)-6-(1,3-dihydro-4-hydroxy-6- methoxy-7-methyl-3-oxoisobenzofuran-5-yl)-4-methyl-4- hexenoat), Methotrexat (CAS-Nr. 59-05-02) oder Mizoribine (CAS-Nr. 50924-49-7) werden nach oraler Applikation im Darm von Patienten resorbiert und führen nach einer kurzzeitigen Erhöhung der Blutspiegel nach der Einnahme (Resorptionspeak) zu konstant hohen Blutspiegeln. Über die Leber und die Gallenflüssigkeit werden die obengenannten Nukleotidsyntheseinhibitoren wieder in den Darm ausgeschieden. Aus dem Darm können die genannten ausgeschiedenen Verbindungen teilweise wieder resorbiert und in das Blut abgegeben werden. Die genannten Verbindungen unterliegen daher der enterohepatische Zirkulation.

Bei dem Einsatz von Nukleotidsyntheseinhibitoren zur Beeinflussung des Immunsystems wurde überraschender Weise gefunden, daß für die erwünschte Wirkung auf das Immunsystem nur kurzzeitige Wirkeffekte dieser Substanzen benötigt werden. Werden Blutspiegel, dieser Substanzen über einen längeren Zeitraum aufrechterhalten, die zu Wirkeffekten führen, so nehmen zwar die Nebenwirkungen zu, die erwünschte Wirkung auf das Immunsystem wird aber nicht gesteigert. Dadurch, daß die Wirkeffekte auf eine kurze Zeitspanne begrenzt werden, läßt sich die Verträglichkeit einer Therapie verbessern bei Aufrechterhaltung der gewünschten pharmakodynamischen Effekte auf das Immunsystem (=verbesserte therapeutische Breite).

Im Falle von Nukeotidsyntheseinhibitoren, die der enterohepatischen Zirkulation unterliegen, kann die Wirkdauer dadurch verkürzt werden, indem Substanzen appliziert werden, welche die enterohepatische Zirkulation unterbrechen. Durch Unterbrechung der enterohepatischen Zirkulation, bleibt die erwünschte Wirkung auf das Immunsystem bestehen, die Nebenwirkungen werden aber drastisch reduziert.

Die obengenannten Nukleotidsyntheseinhibitoren können auch eine verbesserte therapeutische Breite in ihrer Wirkung aufweisen, wenn Verbindungen, die die Wirkung der Nukleotidsyntheseinhibitoren antagonisieren, zeitlich versetzt - also später als die Nukleotidsyntheseinhibitoren - appliziert werden.

Unter dem Begriff therapeutische Breite wird dabei ein Maß für die Verträglichkeit eines Arzneimittels verstanden und ist im wesentlichen der Abstand zwischen der niedrigsten Dosis, die noch zu den erwünschten therapeutischen Effekten führt und der Dosis, die zu Nebenwirkungen führt. Maßstab für die erzielten Verbesserungen sind beispielsweise die Menge an roten Blutkörperchen, Hämoglobingehalt, Hämatokrit, Menge an Glutamat-Oxalacetat-Transaminase, Glutamat-Pyruvat-Transaminase, alkalischer Phosphatase (aus Knochenmark) oder Amylase und das Gewicht im Vergleich mit unbehandelten Patienten.

Die Erfindung betrifft daher eine Zubereitung, enthaltend
1) mindestens eine Verbindung, die die enterohepatische Zirkulation der Nukleotidsyntheseinhibitoren im wesentlichen verhindert aus der Gruppe Colestipol, Colestyramin und Aktivkohle oder eine Verbindung, die zeitlich versetzt die Wirkung der Nukleotidsyntheseinhibitoren antagonisiert aus der Gruppe Uridin, Purin, Purinnukleotide oder Pyrimidinnukleotide, und
2) mindestens einen Nukleotidsyntheseinhibitor aus der Gruppe, 2-Morpholinoethyl-(E)-6-(1,3-dihydro-4-hydroxy-6-methoxy-7-methyl-3-oxoisobenzofuran-5-yl)-4-methyl-4- hexenoat, Mizoribine und Verbindungen der Formeln I oder II 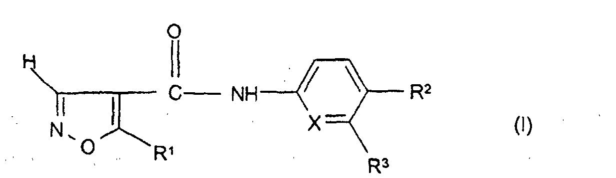 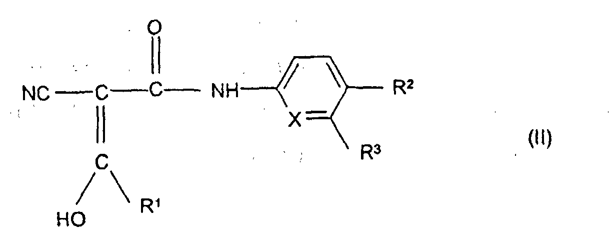 und/oder eine gegebenenfalls stereoisomere Form der Verbindung der Formel I oder 11 und/oder ein physiologisch verträgliches Salz der Verbindung der Formel II, wobei
   - R¹ für: a) (C₁-C₄)-Alkyl,
   b) (C₃-C₅)-Cycloalkyl,
   c) (C₂-C₆)-Alkenyl oder
   d) (C₂-C₆)-Alkinyl, steht,
   - R² für: a) -CF₃,
   b) -O-CF₃,
   c) -S-CF₃,
   d) -OH,
   e) -NO₂,
   f) Halogen,
   g) Benzyl,
   h) Phenyl,
   i) -O-Phenyl,
   k) -CN oder
   l) -O-Phenyl, ein oder mehrfach substituiert mit
      1) (C₁-C₄)-Alkyl,
      2) Halogen,
      3) -O-CF₃ oder
      4) -O-CH₃, steht,
   - R³ für: a) (C₁-C₄)-Alkyl,
   b) Halogen, oder
   c) ein Wasserstoffatom steht, und
   - X für: a) eine -CH-Gruppe oder
   b) ein Stickstoffatom, steht.

Es kann auch eine Mischung der Nukleotidsyntheseinhibitoren und Verbindung der Formel I und II oder Salze der Verbindung der Formel II und eine Mischung der Verbindungen, die die enterohepatische Zirkulation der Verbindung der Formel I oder II im wesentlichen verhindern, eingesetzt werden.

Bevorzugt ist der Einsatz einer Verbindung der Formel I und/oder II und/oder eine gegebenenfalls stereoisomeren Form der Verbindung der Formel I oder II und/oder ein Salz der Verbindung der Formel II, wobei
- R¹ für: a) Methyl,
b) Cyclopropyl oder
c) (C₃-C₅)-Alkinyl steht,
- R² für: -CF₃ oder-CN steht,
- R³ für: ein Wasserstoffatom oder Methyl steht, und
- X für: eine -CH- Gruppe steht,
in Kombination mit mindestens einer Verbindung aus der Gruppe Colestipol, Colestyramin und Aktivkohle.

Insbesondere bevorzugt ist die Verwendung von N-(4-Trifluormethylphenyl)-5-methylisoxazol-4-carboxamid, N-(4-Trifluormethylphenyl)-2-cyan-3-hydroxycrotonsäureamid, 2-Cyano-3-cyclopropyl-3-hydroxy-acrylsäure-(4-cyanophenyl)-amid oder N-(4-Trifluormethylphenyl)-2-cyan-3-hydroxy-hept-2-en-6-incarbonsäureamid in Kombination mit Colestyramin.

Die Herstellung der Verbindung der Formel I oder II erfolgt nach bekannten Verfahren wie sie in EP 484 223; EP 529 500; US 4 061 767; EP 538 783 oder EP 551 230 beschrieben werden. Die Ausgangsstoffe der chemischen Umsetzungen sind bekannt oder lassen sich nach literaturbekannten Methoden leicht herstellen.

Unter dem Begriff Alkyl, Alkenyl oder Alkinyl werden Reste verstanden, deren Kohlenstoffkette gerade oder verzweigt sein kann. Ferner können die Alkenyl- oder Alkinyl-Reste auch mehrere Doppelbindungen beziehungsweise mehrere Dreifachbindungen enthalten. Cyclische Alkylreste sind beispielsweise 3- bis 5-gliedrige Monocyclen wie Cyclopropyl, Cyclobutyl oder Cyclopentyl. Salze der Verbindung der Formel II sind beispielsweise Natrium- oder Lysiniumsalze, die sich wie in der Europäischen Patentanmeldung Nr. EP 0769296 beschrieben herstellen lassen.

Die erfindungsgemäße Zubereitung eignet sich beispielsweise zur Behandlung von
- immunologischer Erkrankungen
- inflammatorischen und zytotoxischen Prozessen im Zusammenhang mit gentherapeutischen Eingriffen
- Krebserkrankungen wie Lungenkrebs, Leukämie, Eierstockkrebs, Sarkome, Kaposi's Sarkom, Meningiom, Darmkrebs, Lymphknotenkrebs, Hirntumore, Brustkrebs, Pankreaskrebs, Prostatakrebs oder Hautkrebs
- Autoimmunerkrankungen wie systemischem Lupus erythematodes oder multipler Sklerose
- Rheumaerkrankungen
- Transplantationen oder Graft-versus-Host-Reaktionen oder Host-versus-Graft-Reaktionen
- Erkrankungen, die durch stark proliferierende Zellen verursacht werden
- Psoriasis oder atypischer Dermatitis
- Allergie, Asthma, Urticaria, Rhinitis oder Uveitis
- Typ II-Diabetes
- zystischer Fibrose, Kolitis, Leberfibrose oder Sepsis
- Chronisch entzündliche Erkrankungen wie Arteriosklerose, Morbus Crohn, Colitis ulcerosa.

Die Erfindung betrifft auch ein Verfahren zur Herstellung der Zubereitung, das dadurch gekennzeichnet ist, daß man die Nukleotidsyntheseinhibitoren und eine Verbindung, die die enterohepatische Zirkulation der Nukleotidsyntheseinhibitoren im wesentlichen verhindert , oder zeitlich versetzt die Wirkung der Nukleotidsyntheseinhibitoren antagonisiert, mit einem pharmazeutisch geeigneten und physiologisch annehmbaren Träger und gegebenenfalls weiteren geeigneten Wirk-, Zusatz- oder Hilfsstoffen in eine geeignete Darreichungsform bringt.

Die erfindungsgemäße Zubereitung kann auch Kompositionen oder Kombinationspackungen umfassen, in denen die Bestandteile nebeneinander gestellt sind und deshalb gleichzeitig, getrennt oder zeitlich abgestuft an ein und denselben menschlichen oder tierischen Körper anwendbar sind. Bevorzugt ist die zeitlich abgestufte Applikation von der Verbindung der Formel I und/oder II vor der Applikation der Verbindung, die die enterohepatische Zirkulation der Verbindung der Formel I oder II im wesentlichen verhindert. Hierzu wird beispielsweise zuerst N-(4-Trifluormethylphenyl)-2-cyan-3-hydroxy-crotonsäureamid (im folgenden als Verbindung 1 bezeichnet) appliziert. Colestyramin, das die enterohepatische Zirkulation der Verbindung 1 im wesentlichen verhindert, wird zeitlich versetzt, also z.B. 2 Stunden oder 4 Stunden nach der Gabe der Verbindung 1, appliziert. Durch diese zeitlich versetzte Gabe der Verbindung 1 und Colestyramin wird die Verbindung 1 zunächst ungehindert aus dem Verdauungstrakt resorbiert.

Nach der Gabe von Colestyramin, welches systemisch nicht resorbiert wird, wird die über die Galle ausgeschiedene Verbindung 1 an Colestyramin gebunden und kann daher nicht wieder reabsorbiert werden; dadurch wird eine Unterbrechung der enterohepatischen Zirkulation bewirkt. Durch diese Maßnahme werden die Wirkungsdauer und die Blutspiegel der Verbindung 1 drastisch reduziert. Trotz dieser drastisch reduzierten Blutspiegel wird die Wirksamkeit im pathologischen Tiermodell, wie der Adjuvans Arthritis bei einer niedrigen, gerade noch wirksamen Dosierung von etwa 2,5 mg/kg/Tag an der Verbindung 1 durch die Gabe von Colestyramin nicht vermindert. Werden im gleichen Tiermodell hohe Dosierungen von 25 mg/kg/Tag der Verbindung 1 eingesetzt, welche bereits zu verschiedenen Nebenwirkungen führen, so beobachtet man durch Gabe von Colestyramin eine deutliche Verminderung der Nebenwirkungen unter Beibehaltung der erwünschten Wirkungen auf das Immunsystem.

Die erfindungsgemäße Zubereitung kann als Dosiereinheit in Form von Arzneiformen wie Kapseln (einschließlich Mikrokapseln, die im allgemeinen keine pharmazeutischen Träger enthalten), Tabletten einschließlich Dragees und Pillen, oder Zäpfchen vorliegen, wobei bei Verwendung von Kapseln das Kapselmaterial die Funktion des Trägers wahrnehmen und der Inhalt z. B. als Pulver, Gel, Lösung Emulsion oder Dispersion vorliegen kann. Besonders vorteilhaft und einfach ist es jedoch, orale oder perorale Formulierungen mit den beiden Wirkstoffkomponenten 1) (z.B. Colestyramin) und 2) (Verbindung der Formel I und/oder II) herzustellen, die die berechneten Mengen der Wirkstoffe zusammen mit jedem gewünschten pharmazeutischen Träger enthalten. Auch eine entsprechende Formulierung (Zäpfchen) für die rektale Therapie kann angewandt werden. Ebenso ist die transdermale Applikation in Form von Salben oder Cremes, parenterale (intraperitoneale, subkutane, intramuskuläre) Injektion oder orale Applikation von Lösungen, die die erfindungsgemäßen Kombinationen enthalten, möglich. Salben, Pasten, Cremes und Puder können neben dem Wirkstoff die üblichen Trägerstoffe enthalten, z. B. tierische und pflanzliche Fette, Wachse, Paraffine, Stärke, Tragant, Cellulosederivate, Polyethylenglykole, Silicone, Kieselsäure, Aluminiumhydroxid, Talkum, Zinkoxid, Milchzucker, Bentonite, Calciumsilikat und Polyamidpulver oder Gemische dieser Stoffe. Die Tabletten, Pillen oder Granulatkörper können nach Verfahren wie Preß-, Tauch- oder Wirbelbettverfahren oder Kesseldragierung hergestellt werden und enthalten Trägermittel und andere übliche Hilfsstoffe wie Gelatine, Agarose, Stärke (z. B. Kartoffel-, Mais- oder Weizenstärke), Cellulose wie Ethylcellulose, Siliziumdioxid, Magnesiumcarbonat, verschiedene Zucker wie Milchzucker und/oder Calciumphosphate. Die Dragierlösung besteht gewöhnlich aus Zucker und/oder Stärkesirup und enthält meistens noch Gelatine, synthetische Celluloseester, Gummi arabicum, Polyvinylpyrrolidon, Pigmente, oberflächenaktive Substanzen, Weichmacher und ähnliche Zusätze entsprechend dem Stand der Technik. Zur Herstellung der Zubereitungsformen kann jedes übliche Fließregulierungs-, Schmier- oder Gleitmittel wie Magnesiumstearat und Trennmittel verwendet werden. Bevorzugt haben die Zubereitungen die Form von Mantel-/Kern-Tabletten oder Mehrschichttabletten, wobei sich die Wirkkomponente 2 im Mantel bzw. im Kern bzw. in einer Schicht befindet, während sich die Wirkkomponente 1 im Kern, im Mantel oder in einer anderen Schicht befindet. Die Wirkstoffkomponenten können auch in retardierter Form vorliegen oder an Retardierungsmaterial adsorbiert bzw. im Retardierungsmaterial (z. B. Cellulose- oder Polystyrolharzbasis, z. B.

Hydroxyethylcellulose) eingeschlossen sein. Eine verzögerte Freisetzung der Wirkstoffe kann auch erreicht werden, indem die betreffende Schicht bzw. das Kompartiment mit üblichen magensaftunlöslichen Überzügen versehen wird.

Bevorzugt ist eine verzögerte Freisetzung der Verbindung, die die enterohepatische Zirkulation der Verbindung der Formel I oder II im wesentlichen verhindert.

Die anzuwendende Dosierung ist selbstverständlich abhängig von verschiedenen Faktoren wie dem zu behandelnden Lebewesen (d. h. Mensch oder Tier), Alter, Gewicht, allgemeiner Gesundheitszustand, dem Schweregrad der Symptome, der zu behandelnden Erkrankung, eventuellen Begleiterkrankungen (falls vorhanden), der Art der begleitenden Behandlung mit anderen Arzneimitteln, oder Häufigkeit der Behandlung. Die Dosierungen werden im allgemeinen mehrfach pro Tag und vorzugsweise einmal bis dreimal pro Tag verabreicht. Die verwendeten Mengen an Einzelwirkstoff orientieren sich hierbei an der empfohlenen Tagesdosis des jeweiligen Einzelwirkstoffs und sollen im allgemeinen im Kombinationspräparat von 10 % bis 300 % der empfohlenen Tagesdosis liegen, bevorzugt von 50 % bis 150 %, insbesondere bei 80 %. Die geeignete Therapie mit den erfindungsgemäßen Kombinationen besteht somit z.B. in der Verabreichung von einer, zwei oder 3 Einzeldosierungen der Zubereitung bestehend aus N-(4-Trifluormethylphenyl)-5-methylisoxazol-4-carboxamid) oder N-(4-Trifluormethylphenyl)-2-cyan-3-hydroxycrotonsäureamid in einer Menge von 2 mg bis 250 mg, bevorzugt 5 mg bis 150 mg, insbesondere 10 mg bis 50 mg, insbesondere bevorzugt 10 mg bis 20 mg und Colestyramin in einer Menge von 250 mg bis 6000 mg, insbesondere von 1500 mg bis 3000 mg.

Femer können die erfindungsgemäßen Zubereitungen auch zusammen mit anderen geeigneten Wirkstoffen, beispielsweise Antiuricopathika, Analgetika, steroidalen oder nichtsteroidalen Antiphlogistika, Thrombocytenaggregationshemmern, Cytokinen, Cytokinagonisten, Cytokinantagonisten oder immunsuppressiven Verbindungen wie Cyclosporin A, FK 506 oder Rapamycin eingesetzt werden.

### Beispiel 1

Adjuvans induzierte Arthritis, Modifikation nach Perper (Proc. Soc. exp. Biol. Med. 137, 506 (1971)) Als Versuchstiere dienten männliche Ratten eines Lewis-Stammes (Moellegard, Dänemark) mit einem Körpergewicht von 160 bis 210 g. Die Tiere erhielten am 1.

Tag eine subkutane Injektion in die Schwanzwurzel mit kompletten Freund'schen Adjuvans, enthaltend eine Mycobacterium butyricum Suspension in schwerem Paraffin Öl (Difco; 6 mg/kg in Paraffin Öl; Merck). Die Verbindungen N-(4-Trifluormethylphenyl)-2-cyan-3-hydroxy-crotonsäureamid und Colestyramin wurden in Carboxymethylcellulose (1 % in Wasser) suspendiert und oral verabreicht. Die Verbindungen wurden einmal täglich vom 1. bis zum 17. Versuchstag appliziert; dann erfolgte die Bestimmung des Pfotenvolumens und Arthritis-Index am 18. Tag.

Die Schwere der Erkrankung wurde durch Messung des Pfotenvolumens beider Hinterpfoten bestimmt. Die Messung erfolgte durch die Wasserverdrängungsmethode mit einem Plethysmometer 2060 (Rhema-Labortechnik, Hofheim, Deutschland). Ferner erfolgte die Bestimmung des Arthritis Index am 18. Tag nach der Injektion.

Bestimmung des Arthritis Index:
- 1. Ohren: 0,5 Punkte für jedes Ohr an dem eine Rötung auftritt und Knoten gebildet werden
- 2. Nase: 1 Punkt für Bindegewebsschwellung
- 3. Schwanz: 1 Punkt für das Auftauchen von Knoten
- 4. Vorderpfoten: 0,5 Punkte für jede Pfote an der wenigsten eine Entzündung an einem Gelenk auftritt
- 5. Hinterpfoten: 1 Punkt für leichte Entzündung (Schwellung) 2 Punkte für eine mittelstarke Entzündung 3 Punkte für eine massive Entzündungsreaktion

Tiere einer Kontrollgruppe "Arthritis Kontrolle" erhielten am 1. Tag eine subkutane Injektion in die Schwanzwurzel mit kompletten Freund'schen Adjuvans und erhielten aber nur das Lösungsmittel (1 % Carboxymethylcellulose in Wasser). Pro Dosierung und in der Kontrollgruppe wurden jeweils 6 Tiere verwendet. Als weitere Kontrollgruppe "gesunde Kontrolle" wurden unbehandelte Tiere eingesetzt. Als Wirkungskriterium diente die Herabsetzung der Pfotenvolumenzunahme und die Abnahme des Arthritis Index gegenüber der unbehandelten Kontrollgruppe und das Gewicht der Tiere jeweils in Prozent und bezogen auf die Arthritis Kontrolle.

In der folgenden Tabelle wird N-(4-Trifluormethylphenyl)-2-cyan-3-hydroxycrotonsäureamid als Verbindung 1 bezeichnet. Colestyramin wurde 4 Stunden später als Verbindung 1 appliziert. Tabelle 1 zeigt die erhaltenen Ergebnisse.

**Tabelle 1**

| | Wirksubstanz (mg/kg Lebendgewicht) | Pfotenvolumen (%) | Arthritis Index (%) | Gewicht (%) |
|---|---|---|---|---|
| gesunde Kontrolle | | | | 30 |
| Arthritis Kontrolle | | | | 18 |
| Colestyramin | 1000 | 35 | 44 | 8 |
| Verbindung 1 | 2,5 | -63 | -77 | 20 |
| Verbindung1 + Colestyramin | 2,5 + 1000 | -70 | -92 | 20 |
| Verbindung 1 | 7,5 | -83 | -92 | 18 |
| Verbindung 1 + Colestyramin | 7,5 + 1000 | -73 | -95 | 27 |
| Verbindung 1 | 25 | -92 | -100 | -2 |
| Verbindung1 + Colestyramin | 25 + 1000 | -72 | -100 | 3 |
| die in der Tabelle gezeigten Werte mit "-" Zeichen geben Abnahme an; alle anderen Werte geben Zunahme im Vergleich zum Anfang des Versuchs an | | | | |

Die mit der erfindungsgemäßen Zubereitung behandelten Tiere zeigten eine Gewichtszunahme, die bei den Mengen 2,5 und 7,5 der Verbindung 1 der gesunden Kontrolle sehr nahe kam und signifikant besser als mit der Verbindung 1 alleine war, während die Wirksamkeit der Verbindung 1 vollständig erhalten blieb.

### Beispiel 2

Die Versuchsbedingungen sind analog zu Beispiel 1. Es wurden die Wirkungen der Verbindung 1 und Colestyramin auf die Menge an roten Blutkörperchen (RBC), Hämoglobingehalt (HGB), Hämatokrit (HCT), Menge an Glutamat-Oxalacetat-Transaminase (GOT) und Glutamat-Pyruvat-Transaminase (GPT) bestimmt.

Colestyramin wurde 4 Stunden später als Verbindung 1 appliziert. Tabelle 2 zeigt die erzielten Ergebnisse.

**Tabelle 2**

| | Wirksubstanz (mg/kg Lebend gewicht) | Pfoten volumen (%) | Arthritis Index (%) | GOT (U/I) | GPT (U/I) | RBC (*10⁶/ mm³) | HGB (g/dl) | HCT (%) | Anzahl der Tiere |
|---|---|---|---|---|---|---|---|---|---|
| gesunde Kontrolle | | | | 50,4 | 23,6 | 7,4 | 13,1 | 38,9 | 4 |
| Arthritis Kontrolle | | | | 50,8 | 20,3 | 7,3 | 13,0 | 38,0 | 6 |
| Colestyramin | 1000 | 10 | 22 | 43,5 | 25,2 | 7,6 | 10,2 | 31,4 | 6 |
| Verbindung 1 | 25 | -92 | -100 | 85,3 | 25,1 | 3,8 | 6,1 | 18,7 | 6 |
| Verbindung 1 + Colestyramin | 25 + 1000 | -72 | -100 | 52,5 | 21,5 | 6,08 | 10,2 | 31,4 | 5 |
| die in der Tabelle gezeigten Werte mit "-" Zeichen geben Abnahme an; alle anderen Werte geben Zunahme im Vergleich zum Anfang des Versuchs an | | | | | | | | | |

Die mit der erfindungsgemäßen Zubereitung behandelten Tiere zeigten eine Normalisierung der Menge an roten Blutkörperchen (RBC), Hämoglobingehalt (HGB), Hämatokrit (HCT), Menge an Glutamat-Oxalacetat-Transaminase (GOT) und Glutamat-Pyruvat-Transaminase (GPT), die der gesunden Kontrolle sehr nahe kam und signifikant besser als mit der Verbindung 1 alleine war, während die Wirksamkeit der Verbindung 1 vollständig erhalten blieb.

### Beispiel 3

Die Versuchsbedingungen sind analog zu Beispiel 1. Es wurde die Wirkungen der Verbindung 1 und Colestyramin auf die Menge an alkalischer Phosphatase (AP) und Amylase bestimmt. Colestyramin wurde 4 Stunden später als Verbindung 1 appliziert. Tabelle 2 zeigt die erzielten Ergebnisse.

**Tabelle 3:**

| | Wirksubstanz (mg/kg Lebend gewicht) | Pfoten volumen (%) | Arthritis Index (%) | AP (U/I) | Amylase (U/I) | Anzahl der getesteten Tiere |
|---|---|---|---|---|---|---|
| gesunde Kontrolle | | | | 312,6 | 3058,3 | 6 |
| Arthritis Kontrolle | | | | 231,5 | 2251,6 | 6 |
| Colestyramin | 1000 | -60 | -46 | 271,8 | 2756,6 | 6 |
| Verbindung 1 | 25 | -110 | -100 | 114,8 | 1306,5 | 6 |
| Verbindung 1 + Colestyramin | 25 + 1000 | -86 | -94 | 206,6 | 2783,3 | 3 |
| die in der Tabelle gezeigten Werte mit"-" Zeichen geben Abnahme an; alle anderen Werte geben Zunahme im Vergleich zum Anfang des Versuchs an | | | | | | |

Die mit der erfindungsgemäßen Zubereitung behandelten Tiere zeigten eine Normalisierung der Menge an Alkalischer Phosphatase, die der gesunden Kontrolle sehr nahe kam und signifikant besser als mit der Verbindung 1 alleine war, während die Wirksamkeit der Verbindung 1 vollständig erhalten blieb.

### Beispiel 4

Eine erfindungsgemäße Zubereitung besteht aus einer kleinen Hartgelatinekapsel die 400 mg Colestyramin enthält und einer größeren Hartgelatinekapsel die 20 mg N-(4-Trifluormethyl-phenyl )-5-methylisoxazol-4-carboxamid enthält. Die kleinere Hartgelatinekapsel wird vollständig von der größeren Kapsel umfaßt. Als Füllmaterial zwischen den beiden Kapseln wird Glucose eingesetzt.

## Patentansprüche

1. Zubereitung, enthaltend
1) mindestens eine Verbindung, die die enterohepatische Zirkulation der Nukleotidsyntheseinhibitoren im wesentlichen verhindert aus der Gruppe Colestipol, Colestyramin und Aktivkohle oder eine Verbindung, die zeitlich versetzt die Wirkung der Nukleotidsyntheseinhibitoren antagonisiert aus der Gruppe Uridin, Purin, Purinnukleotide oder Pyrimidinnukleotide, und
2) mindestens einen Nukleotidsyntheseinhibitor aus der Gruppe 2-Morpholinoethyl-(E)-6-(1,3-dihydro-4-hydroxy-6- methoxy-7-methyl-3-oxoisobenzofuran-5-yl)-4-methyl-4-hexenoat, Mizoribine und Verbindungen der Formeln I oder II 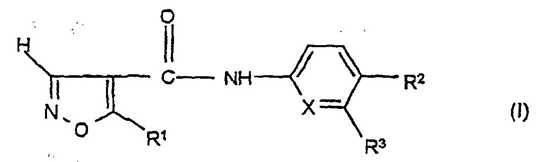 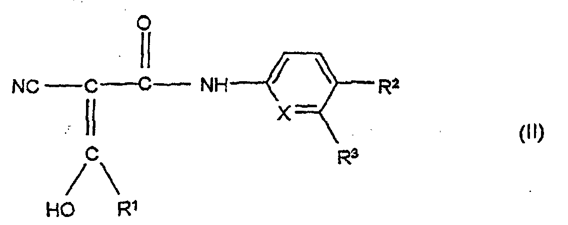 und/oder eine gegebenenfalls stereoisomere Form der Verbindung der Formel I oder II und/oder ein physiologisch verträgliches Salz der Verbindung der Formel II, wobei
R¹ für
a) C₁-C₄-Alkyl,
b) C₃-C₅-Cycloalkyl,
c) C₂-C₆-Alkenyl oder
d) C₂-C₆-Alkinyl, steht,
R² für
a) -CF₃,
b) -O-CF₃,
c) -S-CF₃,
d) -OH,
e) -NO₂,
f) Halogen,
g) Benzyl,
h) Phenyl,
i) -O-Phenyl,
k) -CN oder
l) -O-Phenyl, ein oder mehrfach substituiert mit
1) C₁-C₄-Alkyl,
2) Halogen,
3) -O-CF₃ oder
4) -O-CH₃, steht,
R³ für
a) C₁-C₄-Alkyl,
b) Halogen, oder
c) ein Wasserstoffatom steht, und
X für
a) eine -CH-Gruppe oder
b) ein Stickstoffatom, steht.

2. Zubereitung gemäß Anspruch 1, wobei man eine Verbindung der Formel I und/oder II und/oder eine gegebenenfalls stereoisomeren Form der Verbindung der Formel I oder II und/oder ein Salz der Verbindung der Formel II einsetzt, wobei
R¹ für
a) Methyl,
b) Cyclopropyl oder
c) C₃-C₅-Alkinyl steht,
R² für -CF₃ oder -CN steht,
R³ für ein Wasserstoffatom oder Methyl steht, und
X für eine -CH- Gruppe steht.

3. Zubereitung gemäß der Ansprüche 1 oder 2, wobei man N-(4-Trifluormethylphenyl )-5-methylisoxazol-4-carboxamid als eine Verbindung der Formel I oder N-(4-Trifluormethylphenyl)-2-cyan-3-hydroxy-crotonsäureamid, 2-Cyan-3-cyclopropyl-3-hydroxy-acrylsäure-(4-cyanophenyl)-amid oder N-(4-Trifluormethylphenyl)-2-cyan-3-hydroxy-hept-2-en-6-in-carbonsäureamid als Verbindung der Formel II einsetzt.

4. Zubereitung gemäß einem oder mehreren der Ansprüche 1 bis 3, **dadurch gekennzeichnet, daß** zusätzliche Wirkstoffe aus der Gruppe Antiuricopathika, Analgetika, steroidalen oder nichtsteroidalen Antiphlogistika, Cytokinen, Cytokinagonisten, Thrombocytenaggregationshemmern, Cytokinantagonisten oder immunsuppressiven Verbindungen wie Cyclosporin A, FK 506 oder Rapamycin enthalten sind.

5. Zubereitung gemäß einem oder mehreren der Ansprüche 1 bis 4, **dadurch gekennzeichnet, daß** sie aus Kompositionen oder Kombinationspackungen besteht, in denen die Bestandteile nebeneinander gestellt sind und deshalb gleichzeitig, getrennt oder zeitlich abgestuft an ein und denselben menschlichen oder tierischen Körper anwendbar sind.

6. Zubereitung gemäß Anspruch 5, **dadurch gekennzeichnet, daß** die Applikation der Verbindung der Formel I und/oder II zeitlich vor der Applikation der Verbindung, die die enterohepatische Zirkulation der Verbindung der Formel I oder II im wesentlichen verhindert, durchführbar ist.

7. Verwendung der Zubereitung gemäß einem oder mehreren der Ansprüche 1 bis 6 zur Herstellung eines Arzneimittels zur Behandlung von immunologischen Erkrankungen, inflammatorischen und zytotoxischen Prozessen im Zusammenhang mit gentherapeutischen Eingriffen,
Krebserkrankungen wie Lungenkrebs, Leukämie, Eierstockkrebs, Sarkome, Kaposi's Sarkom, Meningiom, Darmkrebs, Lymphknotenkrebs, Himtumore, Brustkrebs, Pankreaskrebs, Prostatakrebs oder Hautkrebs,
Autoimmunerkrankungen wie systemischem Lupus erythematodes oder multipler Sklerose,
Rheumaerkrankungen,
Transplantationen oder Graft-versus-Host-Reaktionen oder Host-versus-Graft-Reaktionen,
Erkrankungen, die durch stark proliferierende Zellen verursacht werden,
Psoriasis oder atypischer Dermatitis,
Allergie, Asthma, Urticaria, Rhinitis oder Uveitis,
Typ II-Diabetes,
zystischer Fibrose, Kolitis, Leberfibrose oder Sepsis,
Chronisch entzündliche Erkrankungen wie Arteriosklerose, Morbus Crohn oder Colitis ulcerosa.

8. Verfahren zur Herstellung der Zubereitung gemäß der Ansprüche 1 bis 6, **dadurch gekennzeichnet, daß** man mindestens einen Nukleotidsyntheseinhibitor aus der Gruppe 2-Morpholinoethyl-(E)-6-(1,3-dihydro-4-hydroxy-6- methoxy-7-methyl-3-oxoisobenzofuran-5-yl)-4-methyl-4-hexenoat, Mizoribine und Verbindung der Formeln I oder II und eine Verbindung, die die enterohepatische Zirkulation der Nukleotidsyntheseinhibitoren im wesentlichen verhindert aus der Gruppe Colestipol, Colestyramin und Aktivkohle oder
eine Verbindung, die zeitlich versetzt die Wirkung der Nukleotidsyntheseinhibitoren antagonisiert aus der Gruppe Uridin, Purin, Purinnukleotide oder Pyrimidinnukleotide,
mit einem pharmazeutisch geeigneten und physiologisch annehmbaren Träger und gegebenenfalls weiteren geeigneten Wirk-, Zusatz- oder Hilfsstoffen in eine geeignete Darreichungsform bringt.

## Claims

1. A preparation comprising
1) at least one compound which essentially prevents the enterohepatic circulation of the nucleotide synthesis inhibitors from the group consisting of colestipol, colestyramine and active carbon or a compound which antagonizes the action of the nucleotide synthesis inhibitors with a displacement in time from the group consisting of uridine, purine, purine nucleotides or pyrimidine nucleotides, and
2) at least one nucleotide synthesis inhibitor from the group consisting of 2-morpholinoethyl-(E)-6-(1,3-dihydro-4-hydroxy-6-methoxy-7-methyl-3-oxoisobenzofuran-5-yl)-4-methyl-4- hexenoate, mizoribine and compounds of the formulae I or II 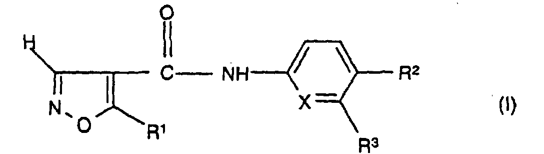 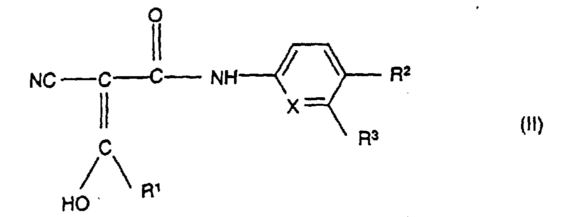 and/or an optionally stereoisomeric form of the compound of the formula I or II and/or a physiologically tolerable salt of the compound of the formula II, where
R¹ is
a) C₁-C₄-alkyl,
b) C₃-C₅-cycloalkyl,
c) C₂-C₆-alkenyl or
d) C₂-C₆-alkynyl,
R² is
a) -CF₃,
b) -O-CF₃,
c) -S-CF₃,
d) -OH,
e) -NO₂,
f) halogen,
g) benzyl,
h) phenyl,
i) -O-phenyl,
k) -CN or
l) -O-phenyl, mono- or polysubstituted by
1) C₁-C₄-alkyl,
2) halogen,
3) -O-CF₃ or
4) -O-CH₃,
R³ is
a) C₁-C₄-alkyl,
b) halogen, or
c) a hydrogen atom, and
X is
a) a -CH group or
c) a nitrogen atom.

2. A preparation as claimed in claim 1, where a compound of the formula I and/or II and/or an optionally stereoisomeric form of the compound of the formula I or II and/or a salt of the compound of the formula II is employed, where
R¹ is
a) methyl,
b) cyclopropyl or
c) C₃-C₅-alkynyl,
R² is -CF₃ or -CN,
R³ is a hydrogen atom or methyl, and
X is a -CH group.

3. A preparation as claimed in claim 1 or 2, where N-(4-trifluoromethyl-phenyl )-5-methylisoxazole-4-carboxamide is employed as a compound of the formula I or N-(4-trifluoromethylphenyl)-2-cyano-3-hydroxycrotonamide, 2-cyano-3-cyclopropyl-3-hydroxyacrylic acid (4-cyanophenyl)amide or N-(4-trifluoromethylphenyl)-2-cyano-3-hydroxyhept-2-en-6-ynecarboxamide is employed as compound of the formula II.

4. A preparation as claimed in one or more of claims 1 to 3, wherein additional active compounds from the group consisting of antiuricopathics, analgesics, steroidal or nonsteroidal antiinflammatory drugs, cytokines, cytokine agonists, platelet aggregation inhibitors, cytokine antagonists or immunosuppressant compounds such as cyclosporin A, FK 506 or rapamycin are contained.

5. A preparation as claimed in one or more of claims 1 to 4, which consists of compositions or combination packs in which the constituents are placed next to one another and can therefore be used simultaneously, separately or sequentially on one and the same human or animal body.

6. A preparation as claimed in claim 5, wherein the administration of the compound of the formula I and/or II can be carried out timewise before the administration of the compound which essentially prevents the enterohepatic circulation of the compound of the formula I or II.

7. The use of the preparation as claimed in one or more of claims 1 to 6 for the production of a pharmaceutical for treating immunological disorders, inflammatory and cytotoxic processes in connection with gene therapy interventions, cancers such as lung cancer, leukemia, ovarian cancer, sarcoma, Kaposi's sarcoma, meningioma, intestinal cancer, lymph node cancer, brain tumors, breast cancer, pancreatic cancer, prostate cancer or skin cancer,
autoimmune disorders such as systemic lupus erythematosus or multiple sclerosis,
rheumatic disorders,
transplantations or graft-versus-host reactions or host-versus-graft reactions,
disorders which are caused by strongly proliferating cells,
psoriasis or atypic dermatitis,
allergy, asthma, urticaria, rhinitis or uveitis, type II diabetes,
cystic fibrosis, colitis, liver fibrosis or sepsis,
chronic inflammatory disorders such as arteriosclerosis, Crohn's disease or ulcerative colitis.

8. A process for the production of the preparation as claimed in claims 1 to 6, which comprises bringing at least one nucleotide synthesis inhibitor from the group consisting of 2-morpholinoethyl-(E)-6-(1,3-dihydro-4-hydroxy-6-methoxy-7-methyl-3-oxoisobenzofuran-5-yl)-4-methyl-4-hexenoate, mizoribine and compound of the formulae I or II and a compound which essentially prevents the enterohepatic circulation of the nucleotide synthesis inhibitors from the group consisting of colestipol, colestyramine and active carbon, or a compound which antagonizes the action of the nucleotide synthesis inhibitors with a displacement in time from the group consisting of uridine, purine, purine nucleotides or pyrimidine nucleotides into a suitable administration form with a pharmaceutically suitable and physiologically acceptable vehicle, and, if appropriate, further suitable active compounds, additives or excipients.

## Revendications

1. Préparation, contenant
1) au moins un composé qui pour l'essentiel empêche la circulation entérohépatique des inhibiteurs de la synthèse des nucléotides, choisi dans le groupe constitué par le colestipol, la colestyramine et le charbon actif, ou un composé qui, avec un décalage dans le temps, antagonise l'effet des inhibitéurs de la synthèse des nucléotides, choisi dans le groupe constitué par l'uridine, la purine, les purine-nucléotides ou les pyrimidine-nucléotides, et
2) au moins un inhibiteur de la synthèse des nucléotides, choisi dans le groupe constitué par le (E)-6-(1,3-dihydro-4-hydroxy-6-méthoxy-7-méthyl-3-oxoisobenzofuranne-5-yl)-4-méthyl-4-hexénoate de 2-morpholinoéthyle, la mizoribine et les composés ayant les formules I ou II 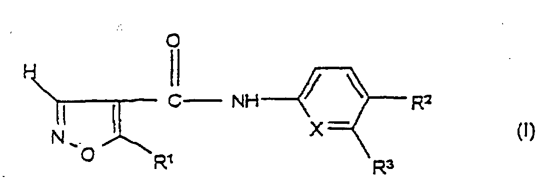 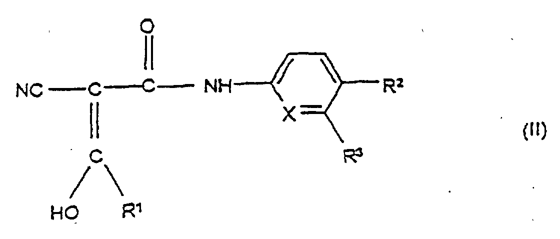 et/ou une forme éventuellement stéréoisomère du composé de formule I ou II, ou un sel physiologiquement compatible du composé de formule II, où
R¹ représente
a) un groupe alkyle en C₁-C₄,
b) un groupe cycloalkyle en C₃-C₅,
c) un groupe alcényle en C₂-C₆, ou
d) un groupe alcynyle en C₂-C₆,
R² représente
a) -CF₃,
b) -O-CF₃,
c) -S-CF₃,
d) -OH,
e) -NO₂,
f) un groupe halogéno,
g) le groupe benzyle,
h) le groupe phényle,
i) le groupe -O-phényle,
k) -CN ou
l) le groupe -O-phényle, une ou plusieurs fois substitué par
1) des groupes alkyle en C₁-C₄,
2) des groupes halogéno,
3) -O-CF₃ ou
4) -O-CH₃,
R³ représente
a) un groupe alkyle en C₁-C₄,
b) un groupe halogéno, ou
c) un atome d'hydrogène, et
X représente
a) un groupe -CH- ou
b) un atome d'azote

2. Préparation selon la revendication 1, dans laquelle on utilise un composé de formule I et/ou II et/ou une forme éventuellement stéréoisomère du composé de formule I ou II et/ou un sel du composé de formule II; où
R¹ représente
a) le groupe méthyle
b) le groupe cyclopropyle, ou
c) un groupe alcynyle en C₃-C₅,
R² représente -CF₃ ou -CN,
R³ représente un atome d'hydrogène ou le groupe méthyle, et
X représente un groupe -CH-.

3. Préparation selon les revendications 1 ou 2, dans laquelle on utilise le N-(4-trifluorométhylphényl)-5-méthylisoxazole-4-carboxamide en tant que composé de formule I, ou du N-(4-trifluorométhylphényl)-2-cyano-3-hydroxy-crotonamide, le (4-cyanophényl)-amide de l'acide 2-cyano-3-cyclopropyl-3-hydroxy-acrylique ou le N-(4-trifluorométhylphényl)-2-cyano-3-hydroxy-hept-2-ène-6-yne-carboxamine en tant que composé de formule II.

4. Préparation selon l'une ou plusieurs des revendications 1 à 3, **caractérisée en ce qu'**elle contient des principes actifs supplémentaires choisis dans le groupe constitué par les antiuricopathiques, les analgésiques, les anti-inflammatoires stéroïdiens ou non-stéroïdiens, les cytokines, les antagonistes des cytokines, les inhibiteurs de l'agrégation thrombocytaire, les antagonistes des cytokines ou les composés immunosuppresseurs tels que la cyclosporine A, le FK 506 ou la rapamycine.

5. Préparation selon l'une ou plusieurs des revendications 1 à 4, **caractérisée en ce qu'**elle est constituée de compositions ou de kits en combinaison, dans lesquels les constituants sont disposés les uns à côté des autres et peuvent donc être utilisés simultanément, séparément ou avec un décalage dans le temps, sur un seul et même organisme humain ou animal.

6. Préparation selon la revendication 5, **caractérisée en ce que** l'administration du composé de formule I et/ou II peut être mise en oeuvre antérieurement à l'administration du composé qui pour l'essentiel empêche la circulation entérohépatique du composé de formule I ou II.

7. Utilisation de la préparation selon l'une ou plusieurs des revendications 1 à 6 pour préparer un médicament destiné au traitement de maladies autoimmunes, de processus inflammatoires et cytotoxiques en liaison avec des interventions en thérapie génique, de maladies oncologiques telles que le cancer des poumons, la leucémie, le cancer des ovaires, les sarcomes, le sarcome de Kaposi, le méningiome, le cancer de l'intestin, le cancer des ganglions lymphatiques, les tumeurs du cerveau, le cancer du sein, le cancer du pancréas, le cancer de la prostate ou le cancer de la peau ; de maladies autoimmunes telles que le lupus érythémateux disséminé ou la sclérose en plaques ; de maladies rhumatismales ; de transplantations ou de réactions greffe contre hôte ou hôte contre greffe ; de maladies qui sont provoquées par des cellules à forte prolifération ; du psoriasis ou de la dermatite atypique ; de l'allergie, de l'asthme, de l'urticaire, de la rhinite ou de l'uvéite ; du diabète de type II ; de la mucoviscidose ; de la colite, de la fibrose hépatique ou de la sepsie ; de maladies inflammatoires chroniques telles que l'artériosclérose, la maladie de Crohn ou la recto-colite hémorragique.

8. Procédé de fabrication de la préparation selon l'une des revendications 1 à 6, **caractérisé en ce qu'**on met sous une forme galénique appropriée au moins un inhibiteur de la synthèse des nucléotides choisis dans le groupe constitué par le (E)-6-(1,3-dihydro-4-hydroxy-6-méthoxy-7-méthyl-3-oxoisobenzofuranne-5-yl)-4-méthyl-4-hexénoate de 2-morpholinoéthyle, la mizoribine et un composé de formules I ou II et un composé qui pour l'essentiel empêche la circulation entérohépatique des inhibiteurs de la synthèse des nucléotides, choisis dans l'ensemble comprenant le colestipol, la colestyramine et le charbon actif, ou un composé qui avec un décalage dans le temps antagonise l'effet des inhibiteurs de la synthèse des nucléotides, choisis dans le groupe constitué par l'uridine, la purine, les purine-nucléotides ou les pyrimidine-nucléotides, en même temps qu'un excipient approprié du point de vue pharmaceutique et physiologiquement acceptable, et éventuellement d'autres principes actifs, additifs ou adjuvants appropriés.
